# EUROPEAN PATENT SPECIFICATION

(11) **EP 4 280 991 B1**
(45) Date of publication and mention of the grant of the patent: **08.01.2025**
(21) Application number: 22702095.5
(22) Date of filing: 11.01.2022
(51) Int. Cl.: A61B 34/00, A61B 34/30, A61B 90/00, A61B 34/37

(54) **SYSTEM AND METHOD FOR TRANSPARENT OVERLAY IN SURGICAL ROBOTIC SYSTEM**
SYSTEM UND VERFAHREN FÜR TRANSPARENTE ÜBERLAGERUNG IN EINEM CHIRURGISCHEN ROBOTERSYSTEM
SYSTÈME ET PROCÉDÉ DE RECOUVREMENT TRANSPARENT DANS UN SYSTÈME ROBOTIQUE CHIRURGICAL

(30) Priority: 19.01.2021 US 202163138891 P
(43) Date of publication of application: 29.11.2023
(73) Proprietor: Covidien LP, Mansfield, MA 02048 (US)
(72) Inventor: KAPADIA, Jaimeen, Boston, Massachusetts 02210 (US)
(74) Representative: Maschio & Soames IP Ltd
(86) International application number: PCT/US2022/011887
(87) International publication number: WO 2022/159301

(56) References cited:
- WO-A1-2020/214193
- US-A1- 2002 135 585
- US-A1- 2017 091 554
- US-A1- 2020 253 673
- JÜRGEN GLATZ: "Real-time intra-operative and endoscopic molecular fluorescence imaging", VOLLSTÄNDIGER ABDRUCK DER VON DER FAKULTÄT FÜR ELEKTROTECHNIK UND INFORMATIONSTECHNIK DER TECHNISCHEN UNIVERSITÄT MÜNCHEN ZUR ERLANGUNG DES AKADEMISCHEN GRADES EINES DOKTOR-INGENIEURS GENEHMIGTEN DISSERTATION, 27 October 2014 (2014-10-27), DE, pages 1 - 175, XP055502704
- GLATZ JÜRGEN: "Real-time intra-operative and endoscopic molecular fluorescence imaging", 27 September 2014 (2014-09-27), pages 1 - 175, XP055908430, Retrieved from the Internet <URL:http://mediatum.ub.tum.de/doc/1230920/1230920.pdf> [retrieved on 20220404]

## Description

### FIELD

The present disclosure generally relates to a surgical robotic systems, and more particularly, to a system and method for generating a transparent overlay for superimposing on a real-time image of the target tissue.

### BACKGROUND

Surgical robotic systems are currently being used in minimally invasive medical procedures. Some surgical robotic systems include a surgical console controlling a surgical robotic arm and a surgical instrument having an end effector (e.g., forceps or grasping instrument) coupled by a wrist assembly to and actuated by the robotic arm.

During surgical procedures with the surgical robotic system the clinician utilizes the surgical console to view a video feed of a surgical site. In most cases, there is a need for the clinician to view other images or data on the surgical console which can impede the viewing of the video feed. Typically, in order to overlay the other images or data onto the video feed, an extra input channel is needed to generate a separate alpha channel for processing alongside the video feed. Thus, there is a need for a surgical robotic system to leverage existing hardware to generate translucent overlays for such images or data to be superimposed on the video feed.

US 2017/091554 provides an image registration device, method, and program that enable easy initial registration between a target object included in a video and a simulation image. A first registration unit performs first registration that is initial registration between an intraoperative video and a simulation image. At this time, a boundary image showing the boundary of the simulation image is displayed on a display so as to be superimposed on the intraoperative video. An operator performs registration between a target object included in the intraoperative video and the boundary image. After the end of the first registration, a second registration unit performs second registration between the simulation image and the target object included in the intraoperative video based on the result of the first registration.

This document further discloses a simulation image that may be generated by defining different colors for the liver and hepatic arteries, hepatic veins, and lesions included in the liver, or that the simulation image may be generated by defining different transparencies. For example, red, blue, and green may be set for hepatic arteries, hepatic veins, and lesions, respectively. In addition, the opacity of the liver may be set to 0.1, the opacity of hepatic arteries and hepatic veins may be set to 0.5, and the opacity of lesions may be set to 0.8.

Further background information can be found in WO 2020/214193 and US 2020/253673.

### SUMMARY

The invention is defined in the appended claims.

In one aspect, a surgical robotic system includes a control tower, a mobile cart coupled to the control tower, and a surgical console coupled to the control tower. The mobile cart includes a surgical robotic arm. The surgical robotic arm includes a surgical instrument and an image capture device. The surgical instrument is actuatable in response to a user input and configured to treat a target tissue. The image capture device is configured to capture a real-time image of the target tissue, and the real-time image includes a plurality of first pixels. The surgical console includes a display, a memory, a user input device, and a controller operably coupled to the display, the memory, and the user input device. The memory is configured to store overlay data. The user input device is configured to generate the user input. The controller is configured to generate an overlay based on the stored overlay data. The overlay includes a plurality of second pixels, wherein each second pixel of the plurality of second pixels includes predetermined color information. The controller is further configured to determine a percentage of transparency of each of the plurality of second pixels based on the predetermined color information; generate an augmented image based on the overlay, the real-time image, and the determined percentage of transparency; and output the augmented image on the display.

In aspects, the overlay data may be a pre-operative image of the target tissue.

In aspects, the generated overlay may be a three-dimensional model of the target tissue based on the stored pre-operative images of the target tissue.

In aspects, the memory may be further configured to store a color lookup table.

In aspects, the color lookup table may be configured to provide a percentage of transparency for each value of the predetermined color information.

In aspects, the controller may be further configured to access the stored color lookup table and return a percentage of transparency based on the predetermined color information.

In aspects, the predetermined color information may be at least one of an RGB or a YUV color code.

In aspects, the controller may be further configured to match each first pixel of the plurality of first pixels of the real-time image with each second pixel of the plurality of second pixels of the overlay.

In aspects, the controller may superimpose each second pixel of the plurality of second pixels of the overlay at the determined percentage of transparency onto each first pixel of the plurality of first pixels of the real-time image.

In another aspect, a surgical robotic system includes a control tower, a mobile cart coupled to the control tower, and a surgical console coupled to the control tower. The mobile cart includes a surgical robotic arm. The surgical robotic arm includes a surgical instrument and an image capture device. The surgical instrument is actuatable in response to a user input and configured to treat a target tissue. The image capture device is configured to capture a real-time image of the target tissue. The surgical console includes a display, a memory, a user input device, and a controller operably coupled to the display, the memory, and the user input device. The memory is configured to store overlay data. The user input device is configured to generate the user input. The controller is configured to: generate an overlay based on the stored overlay data, the overlay includes a plurality of bounding boxes, wherein each bounding box of the plurality of bounding boxes include predetermined color information; determine a percentage of transparency of each bounding box of the plurality of bounding boxes based on the predetermined color information; generate an augmented image based on the overlay, the real-time image, and the determined percentage of transparency; and output the augmented image on the display.

In aspects, the memory may be further configured to store a color lookup table.

In aspects, the color lookup table may be configured to provide a percentage of transparency for each value of the predetermined color information.

In aspects, the controller may be further configured to access the stored color lookup table and return a percentage of transparency based on the predetermined color information.

In aspects, the controller may be further configured to match a position and dimension for each bounding box of the plurality of bounding boxes with a position and dimension of the real-time image.

In aspects, the controller may superimpose each bounding box of the plurality of bounding boxes at the determined percentage of transparency onto the real-time image.

In aspects, the stored overlay data may be at least one of an image, text, or a combination thereof.

In another aspect, the disclosure provides a method of generating a transparent overlay for a real-time image of the target tissue. The method includes: generating an overlay based on stored overlay data, wherein at least one portion of the overlay includes predetermined color information; determining a percentage of transparency of the at least one portion of the overlay based on the predetermined color information; generating an augmented image based on the overlay, the real-time image, and the determined percentage of transparency; and outputting the augmented image on a display.

In aspects, determining the percentage of transparency of at least one portion of the overlay based on the predetermined color information may include accessing a stored color lookup table configured to provide a percentage of transparency for each value of the predetermined color information.

In aspects, accessing the stored color lookup table may include returning a percentage of transparency based on the predetermined color information.

In aspects, the real-time image may include a plurality of first pixels.

In aspects, generating the augmented image based on the overlay, the real-time image, and the determined percentage of transparency may include matching at least one portion of the overlay with the real-time image and superimposing the at least one portion of the overlay at the determined percentage of transparency onto the real-time image.

### BRIEF DESCRIPTION OF THE DRAWINGS

Various aspects of the present disclosure are described herein with reference to the drawings wherein:
FIG. 1 is a schematic illustration of a surgical robotic system including a control tower, a console, and one or more surgical robotic arms according to an aspect of the present disclosure;
FIG. 2 is a perspective view of a surgical robotic arm of the surgical robotic system of FIG. 1;
FIG. 3 is a perspective view of a setup arm with the surgical robotic arm of the surgical robotic system of FIG. 1;
FIG. 4 is a schematic diagram of a computer architecture of the surgical robotic system of FIG. 1;
FIG. 5A is an exemplary view of a real-time image of a target tissue;
FIG. 5B is an exemplary view of an overlay, the overlay including text and image;
FIG. 6 is an exemplary view of an augmented display with the overlay of FIG. 5B transparently superimposed over the real-time image of FIG. 5A;
FIG. 7A is an exemplary view of an overlay, the overlay including a three-dimensional model of a target tissue;
FIG. 7B is an exemplary view of a real-time image of the target tissue; and
FIG. 8 is an exemplary view of an augmented display with the three-dimensional model of FIG. 7A transparently superimposed over the real-time image of FIG. 7B.

### DETAILED DESCRIPTION

The presently disclosed surgical robotic system is described in detail with reference to the drawings, in which like reference numerals designate identical or corresponding elements in each of the several views.

As will be described in detail below, the present disclosure is directed to a surgical robotic system, which includes a surgical console, a control tower, and one or more movable carts having a surgical robotic arm coupled to a setup arm. The control tower includes a controller, which is configured to determine a percentage of transparency for an overlay and superimpose the overlay over a real-time image of a target tissue to prevent occlusion of the underlying real-time image. While the details described below refers to a surgical robotic system, it is to be understood that such disclosure is exemplary and should not be limited to the surgical robotic system. The disclosure may be applied to any suitable surgical system with limited video feed input.

The term "application" may include a computer program designed to perform functions, tasks, or activities for the benefit of a user. Application may refer to, for example, software running locally or remotely, as a standalone program or in a web browser, or other software which would be understood by one skilled in the art to be an application. An application may run on a controller, or on a user device, including, for example, a mobile device, an IOT device, or a server system.

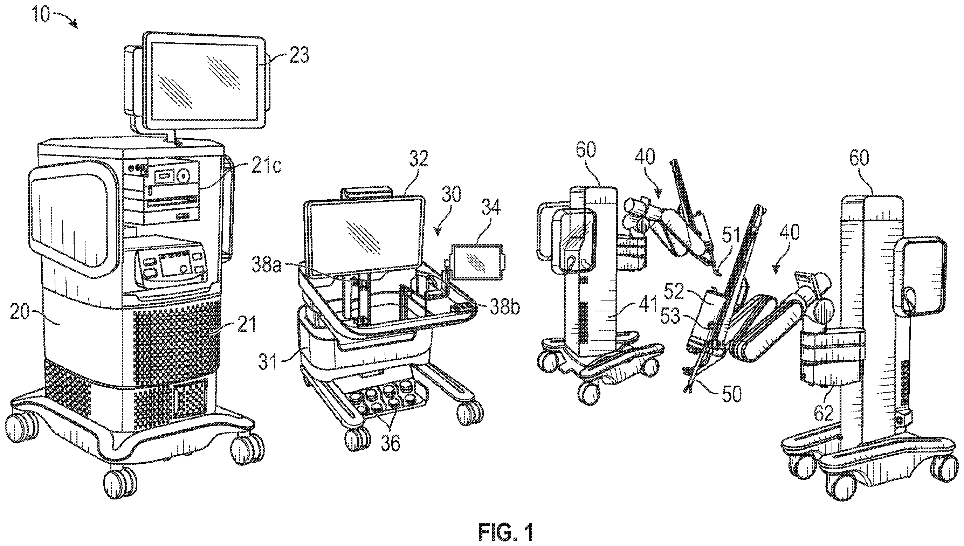

With reference to FIG. 1, a surgical robotic system 10 includes a control tower 20, which is connected to all of the components of the surgical robotic system 10 including a surgical console 30 and one or more robotic arms 40. Each of the robotic arms 40 includes a surgical instrument 50 removably coupled thereto. Each of the robotic arms 40 is also coupled to a movable cart 60.

The surgical instrument 50 is configured for use during minimally invasive surgical procedures. Alternatively, the surgical instrument 50 may be configured for open surgical procedures. In aspects, the surgical instrument 50 may be an endoscope configured to provide a video feed for the user, may be an electrosurgical forceps configured to seal tissue by compression tissue between jaw members and applying electrosurgical current thereto, or may be a surgical stapler including a pair of jaws configured to grasp and clamp tissue whilst deploying a plurality of tissue fasteners, e.g., staples, and cutting stapled tissue.

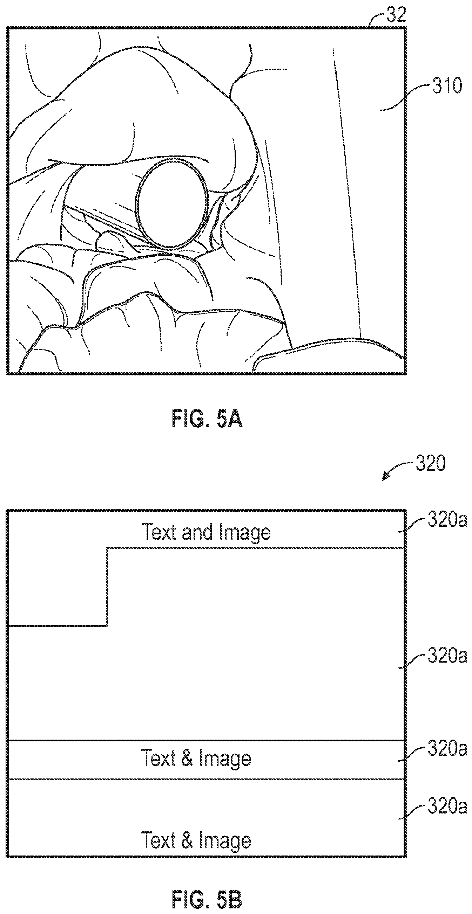

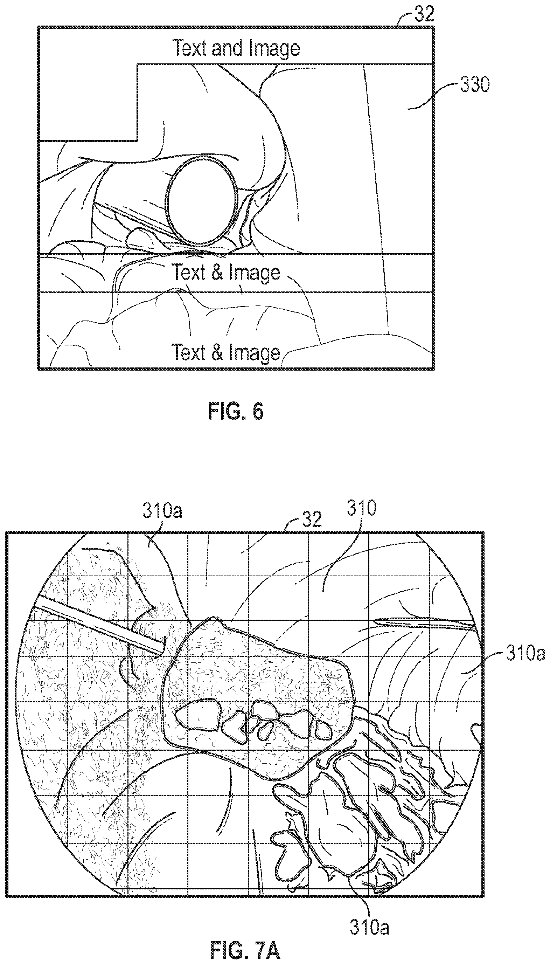

Each of the robotic arms 40 may include an input capture device or camera 51 configured to capture video of a surgical site. The camera 51 may be a stereoscopic camera and may be disposed on the robotic arm 40. The surgical console 30 includes a first display 32, which displays a video feed 310 of the surgical site (FIG. 5A and FIG. 7A) provided by camera 51 disposed on the robotic arms 40, and a second display device 34, which displays a user interface for controlling the surgical robotic system 10.

The surgical console 30 also includes a plurality of user interface devices, such as foot pedals 36 and a pair of handle controllers 38a and 38b which are used by a user to remotely control robotic arms 40, e.g., a teleoperation of the robotic arms 40 via the surgical console 30.

The control tower 20 includes a display 23, which may be a touchscreen, and outputs on the graphical user interfaces (GUIs). The control tower 20 also acts as an interface between the surgical console 30 and one or more robotic arms 40. In particular, the control tower 20 is configured to control the robotic arms 40, such as to move the robotic arms 40 and the corresponding surgical instrument 50, based on a set of programmable instructions and/or input commands from the surgical console 30, in such a way that robotic arms 40 and the surgical instrument 50 execute a desired movement sequence in response to input from the foot pedals 36 and/or the handle controllers 38a and 38b.

Each of the control tower 20, the surgical console 30, and the robotic arm 40 includes a respective computer 21, 31, 41. The computers 21, 31, 41 are interconnected to each other using any suitable communication network based on wired or wireless communication protocols. The term "network," whether plural or singular, as used herein, denotes a data network, including, but not limited to, the Internet, Intranet, a wide area network, or a local area networks, and without limitation as to the full scope of the definition of communication networks as encompassed by the present disclosure. Suitable protocols include, but are not limited to, transmission control protocol/internet protocol (TCP/IP), datagram protocol/internet protocol (UDP/IP), and/or datagram congestion control protocol (DCCP). Wireless communication may be achieved via one or more wireless configurations, e.g., radio frequency, optical, Wi-Fi, Bluetooth (an open wireless protocol for exchanging data over short distances, using short length radio waves, from fixed and mobile devices, creating personal area networks (PANs), ZigBee^{®} (a specification for a suite of high level communication protocols using small, low-power digital radios based on the IEEE 122.15.4-2003 standard for wireless personal area networks (WPANs)).

The computers 21, 31, 41 may include any suitable processor (not shown) operably connected to a memory (not shown), which may include one or more of volatile, non-volatile, magnetic, optical, or electrical media, such as read-only memory (ROM), random access memory (RAM), electrically-erasable programmable ROM (EEPROM), non-volatile RAM (NVRAM), or flash memory. The processor may be any suitable processor (e.g., control circuit) adapted to perform the operations, calculations, and/or set of instructions described in the present disclosure including, but not limited to, a hardware processor, a field programmable gate array (FPGA), a digital signal processor (DSP), a central processing unit (CPU), a microprocessor, and combinations thereof. Those skilled in the art will appreciate that the processor may be substituted for by using any logic processor (e.g., control circuit) adapted to execute algorithms, calculations, and/or set of instructions described herein.

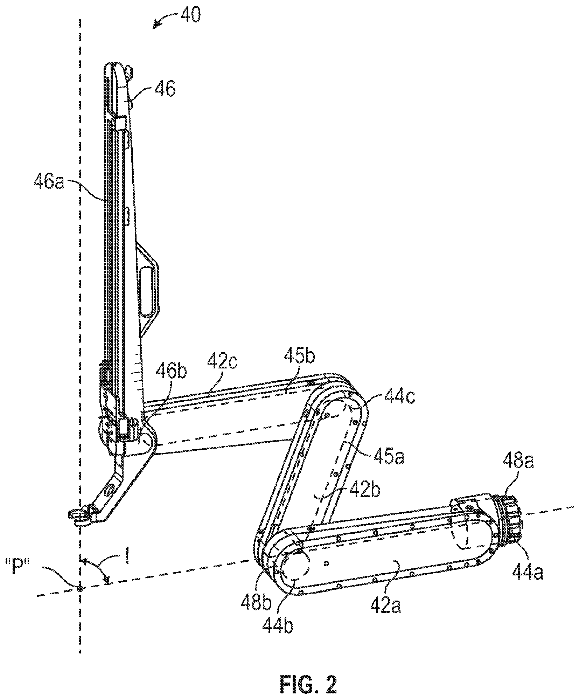

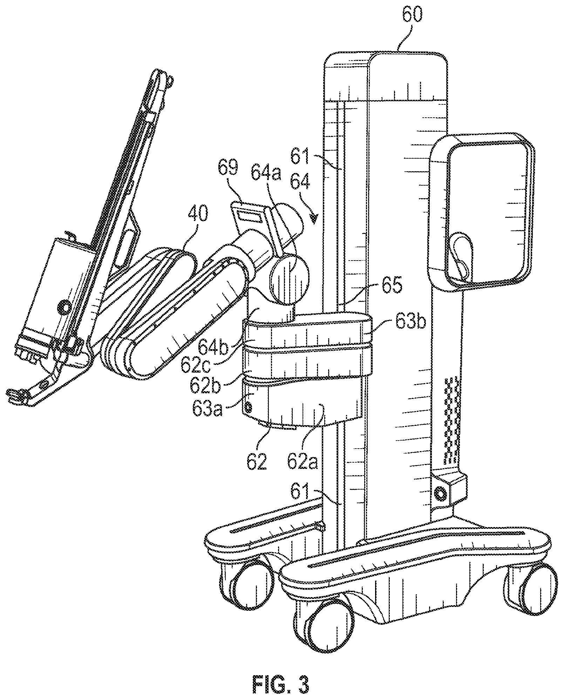

With reference to FIG. 2, each of the robotic arms 40 may include a plurality of links 42a, 42b, 42c, which are interconnected at joints 44a, 44b, 44c, respectively. The joint 44a is configured to secure the robotic arm 40 to the movable cart 60 and defines a first longitudinal axis. With reference to FIG. 3, the movable cart 60 includes a lift 61 and a setup arm 62, which provides a base for mounting of the robotic arm 40. The lift 61 allows for vertical movement of the setup arm 62. The movable cart 60 also includes a display 69 for displaying information pertaining to the robotic arm 40.

The setup arm 62 includes a first link 62a, a second link 62b, and a third link 62c, which provide for lateral maneuverability of the robotic arm 40. The links 62a, 62b, 62c are interconnected at joints 63a and 63b, each of which may include an actuator (not shown) for rotating the links 62b and 62b relative to each other and the link 62c. In particular, the links 62a, 62b, 62c are movable in their corresponding lateral planes that are parallel to each other, thereby allowing for extension of the robotic arm 40 relative to a patient on a surgical table (not shown). The robotic arm 40 may be coupled to the surgical table (not shown). The setup arm 62 includes controls for adjusting movement of the links 62a, 62b, 62c as well as the lift 61.

The third link 62c includes a rotatable base 64 having two degrees of freedom. In particular, the rotatable base 64 includes a first actuator 64a and a second actuator 64b. The first actuator 64a is rotatable about a first stationary arm axis which is perpendicular to a plane defined by the third link 62c and the second actuator 64b is rotatable about a second stationary arm axis which is transverse to the first stationary arm axis. The first and second actuators 64a and 64b allow for full three-dimensional orientation of the robotic arm 40.

The robotic arm 40 also includes a plurality of manual override buttons 53 (FIG. 1) disposed on the IDU 52 and the setup arm 62, which may be used in a manual mode. The clinician may press one or the buttons 53 to move the component associated with the button 53.

With reference to FIG. 2, the robotic arm 40 also includes a holder 46 defining a second longitudinal axis and configured to receive an instrument drive unit (IDU) 52 (FIG. 1). The IDU 52 is configured to couple to an actuation mechanism of the surgical instrument 50 and/or the camera 51 and is configured to move (e.g., rotate) and actuate the instrument 50 and/or the camera 51. IDU 52 transfers actuation forces from its actuators to the surgical instrument 50 to actuate components (e.g., end effectors) of the surgical instrument 50. The holder 46 includes a sliding mechanism 46a, which is configured to move the IDU 52 along the second longitudinal axis defined by the holder 46. The holder 46 also includes a joint 46b, which rotates the holder 46 relative to the link 42c.

The joints 44a and 44b include an actuator 48a and 48b (FIG. 2) configured to drive the joints 44a, 44b, 44c relative to each other through a series of belts 45a and 45b or other mechanical linkages such as a drive rod, a cable, or a lever and the like. In particular, the actuator 48a is configured to rotate the robotic arm 40 about a longitudinal axis defined by the link 42a.

The actuator 48b of the joint 44b is coupled to the joint 44c via the belt 45a, and the joint 44c is in turn coupled to the joint 46c via the belt 45b. Joint 44c may include a transfer case coupling the belts 45a and 45b, such that the actuator 48b is configured to rotate each of the links 42b, 42c and the holder 46 relative to each other. More specifically, links 42b, 42c, and the holder 46 are passively coupled to the actuator 48b which enforces rotation about a remote center point "P" which lies at an intersection of the first axis defined by the link 42a and the second axis defined by the holder 46. Thus, the actuator 48b controls the angle θ between the first and second axes allowing for orientation of the surgical instrument 50. Due to the interlinking of the links 42a, 42b, 42c, and the holder 46 via the belts 45a and 45b, the angles between the links 42a, 42b, 42c, and the holder 46 are also adjusted in order to achieve the desired angle θ. Some or all of the joints 44a, 44b, 44c may include an actuator to obviate the need for mechanical linkages.

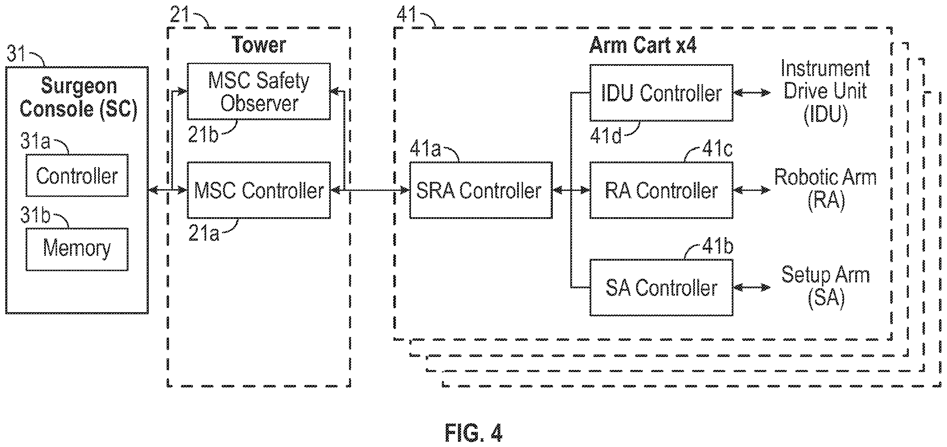

With reference to FIG. 4, each of the computers 21, 31, 41 of the surgical robotic system 10 may include a plurality of controllers, which may be embodied in hardware and/or software. The computer 21 of the control tower 20 includes a controller 21a, the controller 21a receives data from the computer 31 of the surgical console 30 about the current position and/or orientation of the handle controllers 38a and 38b and the state of the foot pedals 36 and other buttons. The controller 21a processes these input positions to determine desired drive commands for each joint of the robotic arm 40 and/or the instrument drive unit 52 and communicates these to the computer 41 of the robotic arm 40. The controller 21a also receives back the actual joint angles and uses this information to determine force feedback commands that are transmitted back to the computer 31 of the surgical console 30 to provide haptic feedback through the handle controllers 38a and 38b.

The computer 41 includes a plurality of controllers, namely, a main cart controller 41a, a setup arm controller 41b, a robotic arm controller 41c, and an instrument drive unit (IDU) controller 41d. The main cart controller 41a receives and processes joint commands from the controller 21a of the computer 21 and communicates them to the setup arm controller 41b, the robotic arm controller 41c, and the IDU controller 41d. The main cart controller 41a also manages instrument exchanges and the overall state of the movable cart 60, the robotic arm 40, and the instrument drive unit 52. The main cart controller 41a also communicates actual joint angles back to the controller 21a.

The setup arm controller 41b controls each of joints 63a and 63b, and the rotatable base 64 of the setup arm 62 and calculates desired motor movement commands (e.g., motor torque) for the pitch axis and controls the brakes. The robotic arm controller 41c controls each joint 44a and 44b of the robotic arm 40 and calculates desired motor torques required for gravity compensation, friction compensation, and closed loop position control of the robotic arm 40. The robotic arm controller 41c calculates a movement command based on the calculated torque. The calculated motor commands are then communicated to one or more of the actuators 48a and 48b in the robotic arm 40. The actual joint positions are then transmitted by the actuators 48a and 48b back to the robotic arm controller 41c.

The IDU controller 41d receives desired joint angles for the surgical instrument 50, such as wrist and jaw angles, and computes desired currents for the motors in the instrument drive unit 52. The IDU controller 41d calculates actual angles based on the motor positions and transmits the actual angles back to the main cart controller 41a.

The robotic arm 40 is controlled as follows. Initially, a pose of the handle controller controlling the robotic arm 40, e.g., the handle controller 38a, is transformed into a desired pose of the robotic arm 40 through a hand eye transform function executed by the controller 21a. The hand eye function, as well as other functions described herein, is/are embodied in software executable by the controller 21a or any other suitable controller described herein. The pose of one of the handle controller 38a may be embodied as a coordinate position and role-pitch-yaw ("RPY") orientation relative to a coordinate reference frame, which is fixed to the surgical console 30. The desired pose of the instrument 50 is relative to a fixed frame on the robotic arm 40. The pose of the handle controller 38a is then scaled by a scaling function executed by the controller 21a. In aspects, the coordinate position is scaled down and the orientation is scaled up by the scaling function. In addition, the controller 21a also executes a clutching function, which disengages the handle controller 38a from the robotic arm 40. In particular, the controller 21a stops transmitting movement commands from the handle controller 38a to the robotic arm 40 if certain movement limits or other thresholds are exceeded and in essence acts like a virtual clutch mechanism, e.g., limits mechanical input from effecting mechanical output.

The desired pose of the robotic arm 40 is based on the pose of the handle controller 38a and is then passed by an inverse kinematics function executed by the controller 21a. The inverse kinematics function calculates angles for the joints 44a, 44b, 44c of the robotic arm 40 that achieve the scaled and adjusted pose input by the handle controller 38a. The calculated angles are then passed to the robotic arm controller 41c, which includes a joint axis controller having a proportional-derivative (PD) controller, the friction estimator module, the gravity compensator module, and a two-sided saturation block, which is configured to limit the commanded torque of the motors of the joints 44a, 44b, 44c.

With continued reference to FIGS. 1, 4, 5B, and 7B, computer 31 of the surgical console 30 further includes a controller 31a and a memory 31b. The memory 31b is configured to store overlay data and a color lookup table.

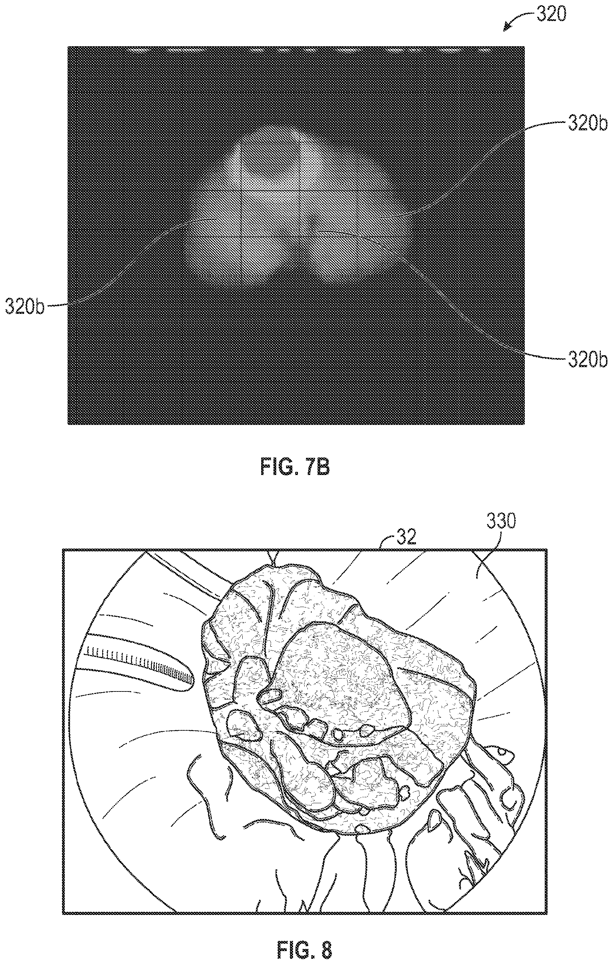

The overlay data includes text or images such as, for example, pre-operative diagnostic images taken of the target tissue at the surgical site (e.g., CT, MRI, fluoroscopic imaging). The controller 31a is configured to receive the stored overlay data and generate an overlay 320. The overlay 320 may be text, images, a three-dimensional model generated from the pre-operative diagnostic images, or any combination thereof (FIG. 5B and 7B).

The overlay 320 is configured to be divided into a plurality of bounding boxes 320a (FIG. 5B) or a plurality of pixels 320b (FIG. 7B). Each bounding box 320a or pixel 320b of overlay 320 is configured to have a predetermined color code and/or color information. The predetermined color information may be a color code derived from RGB or YUV color values, or any other suitable color codes. The color lookup table stored in memory 31b includes a plurality of predefined color codes with a corresponding predefined percentage of transparency. In embodiments, a color code equivalent to black may correspond to a percentage of transparency of 0%, green may correspond to a percentage of transparency of 50%, and orange may correspond to a percentage of transparency of 20%. The controller 31a is further configured to determine the percentage of transparency for each bounding box 320a or pixel 320b of overlay 320. The percentage of transparency is determined by accessing the color lookup table to compare the predetermined color information of the plurality of bounding boxes 320a or pixels 320b of overlay 320 with a predefined color code and return the corresponding predefined percentage of transparency.

With reference to FIGS. 5A, 5B, and 6, when the overlay 320 includes bounding boxes 320a, the controller 31a is configured to match a position and dimension of the plurality of bounding boxes 320a of the overlay 320 (FIG. 5B) with a position and dimension of the video feed 310 of the surgical site (FIG. 5A). Upon matching the plurality of bounding boxes 320a of the overlay 320 with the dimension of the video feed 310, the controller 31a generates an augmented image 330 to be displayed on first display 32. The augmented image 330 is generated by superimposing each bounding box of the plurality of bounding boxes 320a over the video feed 310 based on the determined percentage of transparency.

With reference to FIGS. 7A, 7B, and 8, in the instances, that the overlay 320 includes pixels 320b, the controller 31a is configured to match each of plurality of pixels 320b (FIG. 7B) with a plurality of pixels 310a of the video feed 310 of the surgical site (FIG. 7A). Upon matching the plurality of pixels 320b of the overlay 320 and the plurality of pixels 310a of the video feed 310, the controller 31a generates an augmented image 330. The augmented image 330 is generated by superimposing each pixel of the plurality of pixels 320b of the overlay 320 over each pixel of the plurality of pixels 320b of the video feed 310 based on the determined percentage of transparency.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

## Claims

1. A surgical robotic system (10) comprising:
a control tower (20);
a mobile cart coupled to the control tower, the mobile cart including a surgical robotic arm (40), the surgical robotic arm including:
a surgical instrument (50) actuatable in response to a user input and configured to treat a target tissue; and
an image capture device (51) configured to capture a real-time image of the target tissue, the real-time image including a plurality of first pixels (3 10a); and
a surgical console (30) coupled to the control tower, the surgical console including:
a display (32);
a memory (31b) configured to store overlay data;
a user input device (36, 38a, 38b) configured to generate the user input; and
a controller (31a) operably coupled to the display, the memory, and the user input device, the controller configured to:
generate an overlay (320) based on the stored overlay data, the overlay including a plurality of second pixels (320b), wherein each second pixel of the plurality of second pixels includes predetermined color information;
determine a percentage of transparency of each second pixel of the plurality of second pixels based on the predetermined color information;
generate an augmented image (330) based on the overlay, the real-time image, and the determined percentage of transparency; and
output the augmented image on the display.

2. The surgical robotic system according to claim 1, wherein the overlay data is a pre-operative image of the target tissue, and optionally wherein the generated overlay is a three-dimensional model of the target tissue based on the pre-operative image of the target tissue.

3. The surgical robotic system according to claim 1, wherein the memory is further configured to store a color lookup table.

4. The surgical robotic system according to claim 3, wherein the color lookup table is configured to provide a percentage of transparency for each value of the predetermined color information, and optionally wherein when determining the percentage of transparency of each second pixel of the plurality of second pixels based on the predetermined color information, the controller is further configured to access the stored color lookup table and return a percentage of transparency based on the predetermined color information.

5. The surgical robotic system according to claim 1, wherein the predetermined color information is at least one of an RGB or a YUV color code.

6. The surgical robotic system according to claim 1, wherein the controller is further configured to match each first pixel of the plurality of first pixels of the real-time image with each second pixel of the plurality of second pixels of the overlay, and optionally wherein when generating the augmented image, the controller superimposes each second pixel of the plurality of second pixels of the overlay at the determined percentage of transparency onto each first pixel of the plurality of first pixels of the real-time image.

7. A surgical robotic system (10) comprising:
a control tower (20);
a mobile cart (60) coupled to the control tower, the mobile cart including a surgical robotic arm (40), the surgical robotic arm including:
a surgical instrument (60) actuatable in response to a user input and configured to treat a target tissue; and
an image capture device (51) configured to capture a real-time image of the target tissue; and
a surgical console (30) coupled to the control tower, the surgical console including:
a display (32);
a memory (31b) configured to store overlay data;
a user input device (36, 38a, 38b) configured to generate the user input; and
a controller (31a) operably coupled to the display, the memory, and the user input device, the controller configured to:
generate an overlay (320) based on the stored overlay data, the overlay including a plurality of bounding boxes (320a), wherein each bounding box of the plurality of bounding boxes include a predetermined color information;
determine a percentage of transparency of each bounding box of the plurality of bounding boxes based on the predetermined color information;
generate an augmented image (330) based on the overlay, the real-time image, and the determined percentage of transparency; and
output the augmented image on the display.

8. The surgical robotic system according to claim 7, wherein the memory is further configured to store a color lookup table.

9. The surgical robotic system according to claim 8, wherein the color lookup table is configured to provide a percentage of transparency for each value of the predetermined color information.

10. The surgical robotic system according to claim 9, wherein when determining the percentage of transparency of each bounding box of the plurality of bounding boxes based on the predetermined color information, the controller is further configured to access the stored color lookup table and return a percentage of transparency based on the predetermined color information.

11. The surgical robotic system according to claim 7, wherein the controller is further configured to match a position and dimension for each bounding box of the plurality of bounding boxes with a position and dimension of the real-time image, and optionally wherein when generating the augmented image, the controller superimposes each bounding box of the plurality of bounding boxes at the determined percentage of transparency onto the real-time image.

12. A method of generating a transparent overlay for a real-time image of target tissue, the method comprising:
generating an overlay based on stored overlay data, wherein at least one portion of the overlay includes predetermined color information;
determining a percentage of transparency of the at least one portion of the overlay based on the predetermined color information;
generating an augmented image based on the overlay, the real-time image, and the percentage of transparency; and
outputting the augmented image on a display.

13. The method according to claim 12, wherein determining the percentage of transparency of at least one portion of the overlay based on the predetermined color information includes accessing a stored color lookup table configured to provide the percentage of transparency for each value of the predetermined color information, and optionally wherein accessing the stored color lookup table includes returning a percentage of transparency based on the predetermined color information.

14. The method according to claim 12, wherein generating the augmented image based on the overlay, the real-time image, and the determined percentage of transparency includes matching at least one portion of the overlay with the real-time image and superimposing the at least one portion of the overlay at the determined percentage of transparency onto the real-time image.

15. The method according to claim 12, wherein the at least one portion of the overlay is one of a bounding box or a pixel.

## Patentansprüche

1. Chirurgisches Robotersystem (10), umfassend:
einen Steuerturm (20);
einen mobilen Wagen, der mit dem Steuerturm gekoppelt ist, der mobile Wagen einschließlich eines chirurgischen Roboterarms (40), der chirurgische Roboterarm einschließlich:
eines chirurgischen Instruments (50), das als Reaktion auf eine Benutzereingabe betätigbar ist und konfiguriert ist, um ein Zielgewebe zu behandeln; und
einer Bilderfassungsvorrichtung (51), die konfiguriert ist, um ein Echtzeitbild des Zielgewebes zu erfassen, das Echtzeitbild einschließlich einer Vielzahl von ersten Pixeln (310a); und eine chirurgische Konsole (30), die mit dem Steuerturm gekoppelt ist, die chirurgische Konsole einschließlich:
einer Anzeige (32);
eines Speichers (31b), der konfiguriert ist, um Überlagerungsdaten zu speichern;
einer Benutzereingabevorrichtung (36, 38a, 38b), die konfiguriert ist, um die Benutzereingabe zu erzeugen; und
einer Steuereinrichtung (31a), die mit der Anzeige, dem Speicher und der Benutzereingabevorrichtung wirkgekoppelt ist, wobei die Steuereinrichtung konfiguriert ist zum:
Erzeugen einer Überlagerung (320) basierend auf den gespeicherten Überlagerungsdaten, die Überlagerung einschließlich einer Vielzahl von zweiten Pixeln (320b), wobei jedes zweite Pixel der Vielzahl von zweiten Pixeln vorgegebene Farbinformationen einschließt;
Bestimmen eines Prozentsatzes von Transparenz jedes zweiten Pixels der Vielzahl von zweiten Pixeln basierend auf den vorgegebenen Farbinformationen;
Erzeugen eines erweiterten Bildes (330) basierend auf der Überlagerung, dem Echtzeitbild und dem bestimmten Prozentsatz von Transparenz; und
Ausgeben des erweiterten Bildes auf der Anzeige.

2. Chirurgisches Robotersystem nach Anspruch 1, wobei die Überlagerungsdaten ein präoperatives Bild des Zielgewebes sind, und optional wobei die erzeugte Überlagerung ein dreidimensionales Modell des Zielgewebes basierend auf dem präoperativen Bild des Zielgewebes ist.

3. Chirurgisches Robotersystem nach Anspruch 1, wobei der Speicher ferner konfiguriert ist, um eine Farbnachschlagetabelle zu speichern.

4. Chirurgisches Robotersystem nach Anspruch 3, wobei die Farbnachschlagetabelle konfiguriert ist, um einen Prozentsatz von Transparenz für jeden Wert der vorgegebenen Farbinformationen bereitzustellen, und optional wobei, wenn der Prozentsatz von Transparenz jedes zweiten Pixels der Vielzahl von zweiten Pixeln basierend auf den vorgegebenen Farbinformationen bestimmt wird, die Steuereinrichtung ferner konfiguriert ist, um auf die gespeicherte Farbnachschlagetabelle zuzugreifen und einen Prozentsatz von Transparenz basierend auf den vorgegebenen Farbinformationen zurückzugeben.

5. Chirurgisches Robotersystem nach Anspruch 1, wobei die vorgegebenen Farbinformationen mindestens einer von einem RGB- oder einem YUV-Farbcode sind.

6. Chirurgisches Robotersystem nach Anspruch 1, wobei die Steuereinrichtung ferner konfiguriert ist, um jedes erste Pixel der Vielzahl von ersten Pixeln des Echtzeitbildes mit jedem zweiten Pixel der Vielzahl von zweiten Pixeln der Überlagerung abzugleichen, und optional wobei die Steuereinrichtung, wenn sie das erweiterte Bild erzeugt, jedes zweite Pixel der Vielzahl von zweiten Pixeln der Überlagerung zu dem bestimmten Prozentsatz von Transparenz auf jedes erste Pixel der Vielzahl von ersten Pixeln des Echtzeitbildes legt.

7. Chirurgisches Robotersystem (10), umfassend:
einen Steuerturm (20);
einen mobilen Wagen (60), der mit dem Steuerturm gekoppelt ist, der mobile Wagen einschließlich eines chirurgischen Roboterarms (40), der chirurgische Roboterarm einschließlich:
eines chirurgischen Instruments (60), das als Reaktion auf eine Benutzereingabe betätigbar ist und konfiguriert ist, um ein Zielgewebe zu behandeln; und
einer Bilderfassungsvorrichtung (51), die konfiguriert ist, um ein Echtzeitbild des Zielgewebes zu erfassen; und
eine chirurgische Konsole (30), die mit dem Steuerturm gekoppelt ist, die chirurgische Konsole einschließlich:
einer Anzeige (32);
eines Speichers (31b), der konfiguriert ist, um Überlagerungsdaten zu speichern;
einer Benutzereingabevorrichtung (36, 38a, 38b), die konfiguriert ist, um die Benutzereingabe zu erzeugen; und
einer Steuereinrichtung (31a), die mit der Anzeige, dem Speicher und der Benutzereingabevorrichtung wirkgekoppelt ist, wobei die Steuereinrichtung konfiguriert ist zum:
Erzeugen einer Überlagerung (320) basierend auf den gespeicherten Überlagerungsdaten, die Überlagerung einschließlich einer Vielzahl von Begrenzungsmarkierungen (320a), wobei jede Begrenzungsmarkierung der Vielzahl von Begrenzungsmarkierungen eine vorgegebene Farbinformation einschließt;
Bestimmen eines Prozentsatzes von Transparenz jeder Begrenzungsmarkierung der Vielzahl von Begrenzungsmarkierungen basierend auf den vorgegebenen Farbinformationen;
Erzeugen eines erweiterten Bildes (330) basierend auf der Überlagerung, dem Echtzeitbild und dem bestimmten Prozentsatz von Transparenz; und
Ausgeben des erweiterten Bildes auf der Anzeige.

8. Chirurgisches Robotersystem nach Anspruch 7, wobei der Speicher ferner konfiguriert ist, um eine Farbnachschlagetabelle zu speichern.

9. Chirurgisches Robotersystem nach Anspruch 8, wobei die Farbnachschlagetabelle konfiguriert ist, um einen Prozentsatz von Transparenz für jeden Wert der vorgegebenen Farbinformationen bereitzustellen.

10. Chirurgisches Robotersystem nach Anspruch 9, wobei wenn der Prozentsatz von Transparenz jeder Begrenzungsmarkierung der Vielzahl von Begrenzungsmarkierungen basierend auf den vorgegebenen Farbinformationen bestimmt wird, die Steuereinrichtung ferner konfiguriert ist, um auf die gespeicherte Farbnachschlagetabelle zuzugreifen und einen Prozentsatz von Transparenz basierend auf den vorgegebenen Farbinformationen zurückzugeben.

11. Chirurgisches Robotersystem nach Anspruch 7, wobei die Steuereinrichtung ferner konfiguriert ist, um eine Position und Abmessung für jede Begrenzungsmarkierung der Vielzahl von Begrenzungsmarkierungen mit einer Position und Abmessung des Echtzeitbildes abzugleichen, und optional wobei die Steuereinrichtung, wenn sie das erweiterte Bild erzeugt, jede Begrenzungsmarkierung der Vielzahl von Begrenzungsmarkierungen zu dem bestimmten Prozentsatz von Transparenz auf das Echtzeitbild legt.

12. Verfahren zum Erzeugen einer transparenten Überlagerung für ein Echtzeitbild eines Zielgewebes, das Verfahren umfassend:
Erzeugen einer Überlagerung basierend auf gespeicherten Überlagerungsdaten, wobei mindestens ein Abschnitt der Überlagerung vorgegebene Farbinformationen einschließt;
Bestimmen eines Prozentsatzes von Transparenz des mindestens einen Abschnitts der Überlagerung basierend auf den vorgegebenen Farbinformationen;
Erzeugen eines erweiterten Bildes basierend auf der Überlagerung, dem Echtzeitbild und dem Prozentsatz von Transparenz; und
Ausgeben des erweiterten Bildes auf einer Anzeige.

13. Verfahren nach Anspruch 12, wobei das Bestimmen des Prozentsatzes von Transparenz mindestens eines Abschnitts der Überlagerung basierend auf der vorgegebenen Farbinformationen das Zugreifen auf eine gespeicherte Farbnachschlagetabelle einschließt, die konfiguriert ist, um den Prozentsatz von Transparenz für jeden Wert der vorgegebenen Farbinformationen bereitzustellen, und optional wobei das Zugreifen auf die gespeicherte Farbnachschlagetabelle das Zurückgeben eines Prozentsatzes von Transparenz basierend auf den vorgegebenen Farbinformationen einschließt.

14. Verfahren nach Anspruch 12, wobei das Erzeugen des erweiterten Bildes basierend auf der Überlagerung, dem Echtzeitbild und dem bestimmten Prozentsatz von Transparenz das Abgleichen mindestens eines Abschnitts der Überlagerung mit dem Echtzeitbild und das Legen des mindestens einen Abschnitts der Überlagerung zu dem bestimmten Prozentsatz von Transparenz auf das Echtzeitbild einschließt.

15. Verfahren nach Anspruch 12, wobei der mindestens eine Abschnitt der Überlagerung eines von einer Begrenzungsmarkierung oder einem Pixel ist.

## Revendications

1. Système robotique chirurgical (10) comprenant :
une tour de commande (20) ;
un chariot mobile couplé à la tour de commande, le chariot mobile comportant un bras robotique chirurgical (40), le bras robotique chirurgical comportant :
un instrument chirurgical (50) actionnable en réponse à une entrée utilisateur et configuré pour traiter un tissu cible ; et
un dispositif de capture d'images (51) configuré pour capturer une image en temps réel du tissu cible, l'image en temps réel comportant une pluralité de premiers pixels (310a) ; et une console chirurgicale (30) couplée à la tour de commande, la console chirurgicale comportant :
un affichage (32) ;
une mémoire (31b) configurée pour stocker les données de superposition ;
un dispositif d'entrée utilisateur (36, 38a, 38b) configuré pour générer l'entrée utilisateur ; et
un dispositif de commande (31a) couplé de manière opérationnelle à l'affichage, à la mémoire et au dispositif d'entrée utilisateur, le dispositif de commande étant configuré pour :
générer une superposition (320) en fonction des données de superposition stockées, la superposition comportant une pluralité de seconds pixels (320b), dans lequel chaque second pixel de la pluralité de seconds pixels comporte des informations de couleur prédéterminées ;
déterminer un pourcentage de transparence de chaque second pixel de la pluralité de seconds pixels en fonction des informations de couleur prédéterminées ;
générer une image augmentée (330) en fonction de la superposition, de l'image en temps réel et du pourcentage de transparence déterminé ; et
afficher l'image augmentée sur l'affichage.

2. Système robotique chirurgical selon la revendication 1, dans lequel les données de superposition sont une image préopératoire du tissu cible, et éventuellement dans lequel la superposition générée est un modèle tridimensionnel du tissu cible en fonction de l'image préopératoire du tissu cible.

3. Système robotique chirurgical selon la revendication 1, dans lequel la mémoire est en outre configurée pour stocker une table de consultation de couleurs.

4. Système robotique chirurgical selon la revendication 3, dans lequel la table de consultation de couleurs est configurée pour fournir un pourcentage de transparence pour chaque valeur des informations de couleur prédéterminées, et éventuellement dans lequel, lors de la détermination du pourcentage de transparence de chaque second pixel de la pluralité de seconds pixels en fonction des informations de couleur prédéterminées, le dispositif de commande est en outre configuré pour accéder à la table de consultation de couleurs stockée et renvoyer un pourcentage de transparence en fonction des informations de couleur prédéterminées.

5. Système robotique chirurgical selon la revendication 1, dans lequel les informations de couleur prédéterminées sont au moins l'un parmi un code couleur RVB ou YUV.

6. Système robotique chirurgical selon la revendication 1, dans lequel le dispositif de commande est en outre configuré pour faire correspondre chaque premier pixel de la pluralité de premiers pixels de l'image en temps réel avec chaque second pixel de la pluralité de seconds pixels de la superposition, et éventuellement dans lequel, lors de la génération de l'image augmentée, le dispositif de commande superpose chaque second pixel de la pluralité de seconds pixels de la superposition au pourcentage de transparence déterminé sur chaque premier pixel de la pluralité de premiers pixels de l'image en temps réel.

7. Système robotique chirurgical (10) comprenant :
une tour de commande (20) ;
un chariot mobile (60) couplé à la tour de commande, le chariot mobile comportant un bras robotique chirurgical (40), le bras robotique chirurgical comportant :
un instrument chirurgical (60) actionnable en réponse à une entrée utilisateur et configuré pour traiter un tissu cible ; et
un dispositif de capture d'images (51) configuré pour capturer une image en temps réel du tissu cible ; et
une console chirurgicale (30) couplée à la tour de commande, la console chirurgicale comportant :
un affichage (32) ;
une mémoire (31b) configurée pour stocker les données de superposition ;
un dispositif d'entrée utilisateur (36, 38a, 38b) configuré pour générer l'entrée utilisateur ; et
un dispositif de commande (31a) couplé de manière opérationnelle à l'affichage, à la mémoire et au dispositif d'entrée utilisateur, le dispositif de commande étant configuré pour :
générer une superposition (320) en fonction des données de superposition stockées, la superposition comportant une pluralité de boîtes de délimitation (320a), dans lequel chaque boîte de délimitation de la pluralité de boîtes de délimitation comporte une information de couleur prédéterminée ;
déterminer un pourcentage de transparence de chaque boîte de délimitation de la pluralité de boîtes de délimitation en fonction des informations de couleur prédéterminées ;
générer une image augmentée (330) en fonction de la superposition, de l'image en temps réel et du pourcentage de transparence déterminé ; et
afficher l'image augmentée sur l'affichage.

8. Système robotique chirurgical selon la revendication 7, dans lequel la mémoire est en outre configurée pour stocker une table de consultation de couleurs.

9. Système robotique chirurgical selon la revendication 8, dans lequel la table de consultation de couleurs est configurée pour fournir un pourcentage de transparence pour chaque valeur des informations de couleur prédéterminées.

10. Système robotique chirurgical selon la revendication 9, dans lequel, lors de la détermination du pourcentage de transparence de chaque boîte de délimitation de la pluralité de boîtes de délimitation en fonction des informations de couleur prédéterminées, le dispositif de commande est en outre configuré pour accéder à la table de consultation de couleurs stockée et renvoyer un pourcentage de transparence en fonction des informations de couleur prédéterminées.

11. Système robotique chirurgical selon la revendication 7, dans lequel le dispositif de commande est en outre configuré pour faire correspondre une position et une dimension pour chaque boîte de délimitation de la pluralité de boîtes de délimitation avec une position et une dimension de l'image en temps réel, et éventuellement dans lequel, lors de la génération de l'image augmentée, le dispositif de commande superpose chaque boîte de délimitation de la pluralité de boîtes de délimitation au pourcentage de transparence déterminé sur l'image en temps réel.

12. Procédé de génération d'une superposition transparente pour une image en temps réel d'un tissu cible, le procédé comprenant :
la génération d'une superposition en fonction des données de superposition stockées, dans lequel au moins une partie de la superposition comporte des informations de couleur prédéterminées ;
la détermination d'un pourcentage de transparence de l'au moins une partie de la superposition en fonction des informations de couleur prédéterminées ;
la génération d'une image augmentée en fonction de la superposition, de l'image en temps réel et du pourcentage de transparence ; et
l'affichage de l'image augmentée sur un affichage.

13. Procédé selon la revendication 12, dans lequel la détermination du pourcentage de transparence d'au moins une partie de la superposition en fonction des informations de couleur prédéterminées comporte l'accès à une table de consultation de couleur stockée configurée pour fournir le pourcentage de transparence pour chaque valeur des informations de couleur prédéterminées, et éventuellement dans lequel l'accès à la table de consultation de couleur stockée comporte le retour d'un pourcentage de transparence en fonction des informations de couleur prédéterminées.

14. Procédé selon la revendication 12, dans lequel la génération de l'image augmentée en fonction de la superposition, de l'image en temps réel et du pourcentage de transparence déterminé comporte la mise en correspondance d'au moins une partie de la superposition avec l'image en temps réel et la superposition de l'au moins une partie de la superposition au pourcentage de transparence déterminé sur l'image en temps réel.

15. Procédé selon la revendication 12, dans lequel l'au moins une partie de la superposition est l'un parmi une boîte de délimitation ou un pixel.
